(19) Europäisches Patentamt
European Patent Office
Office européen des brevets

(11) Numéro de publication : **0 494 567 A1**

(12) # DEMANDE DE BREVET EUROPEEN

(21) Numéro de dépôt : **91403564.7**

(51) Int. Cl.$^5$ : **C07C 45/49,** C07C 47/52

(22) Date de dépôt : **30.12.91**

(30) Priorité : **31.12.90 FR 9016531**

(43) Date de publication de la demande :
**15.07.92 Bulletin 92/29**

(84) Etats contractants désignés :
**AT BE CH DE DK ES FR GB GR IT LI LU NL SE**

(71) Demandeur : **RHONE-POULENC CHIMIE**
**25, quai Paul Doumer**
**F-92408 Courbevoie Cédex (FR)**

(72) Inventeur : **Krumenacker, Léon**
**2, Allée du Bois Rond**
**F-69360 Serezin du Rhone (FR)**
Inventeur : **Metz, François**
**6, rue de la Salle des Fêtes**
**F-69390 Vernaison (FR)**
Inventeur : **Leconte, Philippe**
**30 rue du Moenchsberg**
**F-68100 Mulhouse (FR)**

(74) Mandataire : **Ricalens, François et al**
**RHONE-POULENC CHIMIE Service Brevets**
**Chimie 25, quai Paul Doumer**
**F-92408 Courbevoie (FR)**

(54) **Réactif et procédé d'hydrocarbonylation triphasique.**

(57) L'invention concerne un réactif d'Hydrocarbonylation de dérivé insaturés caractérisé par le fait qu'il comporte : une phase aqueuse contenant au moins une espèce chimique stable à caractère basique ; une phase organique contenant un catalyseur comportant au moins un élément de la colonne VIII de la classification périodique des éléments, ledit élément de la colonne VIII de la classification périodique complexé avec au moins un agent de coordination (ou ligand) de préférence une pnictine ; une phase vapeur contenant de l'hydrogène et du monoxyde de carbone. La pression partielle de chacun de ces deux gaz étant au moins égale à $10^4$ MPa.

L'invention concerne un procédé de préparation d'aldéhydes insaturés dans lequel on fait réagir le réactif précité avec un dérivé halogéné.

EP 0 494 567 A1

La présente invention a pour objet un réactif et un procédé d'hydrocarbonylation de dérivés insaturés.Elle vise plus particulièrement une réaction d'hydrocarbonylation de dérivés halogénés ou pseudo-halogénés au moyen d'un réactif ayant 2 phases liquides.

Le Brevet US 3 960 932 décrit un procédé général de préparation d'aldéhydes, par réaction d'halogénures d'aryle, de vinyle ou de composés hétérocycliques, avec un mélange de monoxyde de carbone et d'hydrogène, en présence d'une amine tertiaire et d'un catalyseur au palladium consistant en un complexe d'un dérivé du palladium divalent avec une phosphine, un phosphite ou une arsine ou en l'association d'un sel de palladium divalent ou de palladium métallique finement divisé avec un agent complexant du groupe des phosphines, des phosphites ou des arsines. Les halogénures d'aryle utilisés dans le procédé selon US 3 960 932 sont des bromures ou des iodures de phényle ou de naphtyle, non substitués ou substitués par des groupements alkyle, alcoxyle, nitrile ou carboxylate d'alkyle.

Le Brevet européen EP 109 606 propose d'augmenter la cinétique de la réaction d'hydrocarbonylation du précédent procédé, en opérant sous des pressions de 2 à 40 MPa (20 à 400 bar), à des températures de 80°C à 250°C et en utilisant des quantités importantes de phosphine ou de phosphite (2 à $10^5$ fois la quantité molaire de catalyseur).

Il faut observer que ces procédés de l'art antérieur n'utilisent que des substrats très réactifs tels que bromures et iodures. Les réactifs utilisés ne sont pas assez puissants pour permettre l'utilisation de substrats du type chlorure d'aryle.

Les substrats du type chlorure sont pourtant particulièrement intéressants car ils sont faciles à synthétiser et sont peu coûteux.

C'est pourquoi, un des buts de la présente invention est de fournir un réactif permettant de réaliser l'hydrocarbonylation des dérivés halogénés avec une cinétique suffisamment importante pour que l'on puisse utiliser comme substrat des dérivés chlorés ou des pseudo-halogénés.

Un autre but de la présente invention est de fournir un procédé qui permette le recyclage des systèmes catalytiques et notamment des métaux nobles utilisés et des agents coordinants telles que les phosphines. Un autre but de la présente invention est de fournir un procédé qui permette l'utilisation de faibles quantités de métaux nobles pour une cinétique élevée.

Un autre but de la présente invention est de fournir un procédé et un réactif qui permette d'utiliser des composés chimiques de faibles prix.

Ces buts et d'autres qui apparaitront par la suite sont atteints au moyen d'un réactif d'hydrocarbonylation de dérivés insaturés comportant:

a) une phase aqueuse contenant au moins une espèce chimique stable à caractère basique ;

b) une phase organique contenant un catalyseur comportant au moins un élément de la colonne VIII de la classification périodique des éléments, ledit élément de la colonne VIII de la classification périodique complexé avec au moins un agent de coordination (ou ligand);

c) une phase vapeur contenant de l'hydrogène et du monoxyde de carbone ; la pression partielle de chacun de ces deux gaz étant au moins égale à $10^4$ Pa.

Lesdits agents de coordination sont avantageusement des dérivés hydrocarbonés des éléments de la colonne V. Lesdits dérivés hydrocarbonés des éléments de la colonne V dérivent de l'état de valence III de l'azote telles les amines, du phosphore telles les phosphines, de l'arsenic telles les arsines et de l'antimoine telles les stibines ; ces composés seront, par analogie avec le terme de pnicture, désignés dans la présente description, sous le terme de pnictines.

Ils sont avantageusement choisis parmi les dérivés hydrocarbonés des éléments de la colonne VB (Cf. Bull, Soc. Chim. Fr. Supp. au N° 1 Janvier 1966), de préférence de période supérieure à la 2e, avantageusement du phosphore tels que les phosphines.

Avantageusement ledit catalyseur comporte comme pnictine, une trialcoylphosphine, de préférence une triphénylphosphine. Ladite phosphine et ledit métal de la colonne VIII sont avantageusement sous forme de tétrakis phosphine métal.

Dans la présente description le terme alcoyle est pris dans le sens du dictionnaire DUVAL Presse Scientifique Internationale de PARIS VI 1959.

Dans la suite de la description, on ne se refèrera plus qu'aux phosphines qui sont les plus utilisées ; les phosphines joueront le rôle de paradigme vis-à-vis des stibines et des arsines. Cet enseignement par l'exemple ne saurait être en aucun cas limitatif.

Les phosphines préférées sont les phosphines qui sont les meilleurs activateurs des métaux du groupe VIII, notamment les phosphines très basiques telles que les phosphines de tricyclohexyle. Il peut être toutefois plus intéressant notamment en raison de leur prix et en raison de leur stabilité, d'utiliser des phosphines de triaryle telles que les triphénylphosphines.

La quantité d'eau présente dans le réactif doit être telle qu'il y a séparation en 2 phases et de préférence

que la phase aqueuse représente au moins 1/10e du volume de la phase organique. Le rapport des phases organiques et aqueuses ne jouent qu'un rôle économique et devra être adapté dans chaque cas particulier, mais il préférable que la phase aqueuse soit capable de dissoudre la quasi-totalité de l'espèce chimique à caractère basique.

Il est préférable que le coefficient de partage des bases entre la phase aqueuse et la phase organique soit tel que le rapport entre la fraction présente dans la phase aqueuse et celle présente dans la phase organique soit au moins égal à 10% avantageusement à 50 % de préférence 90 %.

Lorsque le substrat et/ou le produit d'arrivée sont peu solubles en phase aqueuse, il est possible de mener la réaction, en un système multiphasé, soit en ajoutant un solvant A.

Les solvants A sont des solvants organiques choisis de manière qu'ils dissolvent au moins 1 %, de préférence au moins 2 %, en masse du substrat et sont suffisamment hydrophobes pour n'être pas miscibles à l'eau en toute proportion.

Il est préférable que l'eau ne puisse dissoudre qu'au plus 10 % de solvant A, avantageusement au plus 1 %, en masse et ce même en présence du substrat en tant que tiers solvant.

Il est préférable que le solvant A ne puisse dissoudre qu'au plus 10 % d'eau, avantageusement au plus 1 %, en masse et ce même en présence du substrat en tant que tiers solvant.

Les solvants A peuvent être des mélanges, y compris des fractions pétrolières. Naturellement, dans les conditions opératoires les solvants A doivent être inertes vis-à-vis des substrats et des réactifs utilisés.

Les familles préférées de solvants sont choisies dans le groupe constitué par les hydrocarbures, les dérivés aromatiques, les éthers, les esters et les solvants halogénés. Ces solvants doivent être moins sensiblement moins réactifs que les substrats, de manière à ne pas interférer avec la réaction à moins que le substrat soit en excès pour jouer un rôle de solvant.

A titre de paradigmes de ces familles, on peut citer comme dérivés aliphatiques halogénés le dichlorométhane, le dichloro-1,2-éthane, le trichloro-1,1,1- éthane, comme dérivés aromatiques le toluène et comme dérivés aromatiques halogénés le chlorobenzène, comme esters l'acétate d'éthyle et l'acétate d'isopropyle, comme éthers, l'oxyde de tertio-butyle et de méthyle ainsi que l'anisole et les alcools lourds, c'est-à-dire répondant aux contraintes d'immiscibilité comme spécifié ci-dessus.

Pour des raisons d'économie industrielle, il est préférable que le solvant A soit distillable sous pression atmosphérique ou sous vide primaire ou secondaire. Le rôle de l'espèce chimique stable à caractère basique est très important et détermine en partie le rendement de transformation en aldéhydes et le rapport aldéhyde sur acide.

Ainsi, selon la présente invention, il a été montré que lorsque l'on choisissait une base dont le pKa de l'acide associé était au moins égal à 10, la quantité relative d'acide restait importante. Lorsque l'on choisissait une base dont le pKa de l'acide associé était compris entre 4 et 10, la proportion d'acide était significativement diminuée, cependant que la réaction parasite principale devenait une réaction de déshalogénation réductrice pour donner un composé ou l'halogénure était remplacé par un hydrogène.

Un acide dont le pKa est au plus égal à 4 permet de favoriser la réaction d'hydrocarbonylation donnant naissance à un aldéhyde vis-à-vis des 2 réactions parasites, la réaction de réduction et la réaction d'hydroxycarbonylation pour donner un acide.

Ainsi dans la grande majorité des cas, le domaine préféré est un domaine dans lequel on utilise une base dont l'acide associé présente un pKa au plus égal à 4, de préférence au plus égal à 2.

Toutefois, ce domaine préféré n'exclue pas l'utilisation d'autres bases. En effet, d'une part il est possible d'utiliser des bases fortes en les introduisant progressivement dans la phase aqueuse de manière à maîtriser le pH de cette dernière.

Les quantités utilisées d'espèce(s) chimique(s) à caractère basique sont telles que le pH désiré soit obtenu pendant la plus grande partie de la réaction au moins ; ainsi il est préférable que la quantité d'espèce(s) chimique(s) à caractère basique soit au moins égale à la quantité stoechiométriquement nécessaire pour "neutraliser" l'acide halohydrique dégagé par la réaction d'hydrocarbonylation.

D'autre part, certains aldéhydes n'ont des domaines de stabilité que dans certaines zones de pH, de sorte qu'il n'est possible d'exclure à priori aucune zone de pH de la phase aqueuse du réactif et ce, bien qu'il soit souhaitable que le potentiel hydrogène soit régulé à des valeurs inférieures à 4, de préférence à 2.

L'utilisation d'une phase aqueuse présente de nombreux avantages parmi lesquels on peut citer la possibilité d'utiliser des réactifs pièges d'aldéhydes tels que par exemple les ions sulfites pour donner des combinaisons bi-sulfitiques.

La phase aqueuse permet également de récupérer plus aisément les aldéhydes solubles dans l'eau. Dans ces cas là, il est même possible de prévoir une récupération en continu ou en semi-continue des aldéhydes formés par sou tirage en cours de réaction d'une fraction ou d'un flux de la phase aqueuse de la récupération de l'aldéhyde formé et de la réintroduction de la phase aqueuse dans le milieu réactionnel.

Le fait que l'essentiel du système catalytique, ou du moins sa partie la plus coûteuse, à savoir la partie pnictine et métaux nobles, se trouve dans la phase organique, permet une récupération et un recyclage plus aisé des éléments de la colonne VIII du tableau périodique des éléments ainsi que des pnictines en général des phosphines.

On obtient ainsi une phase organique qui contient un métal de la colonne VIII complexé par des composés tels que les phosphines et donc susceptibles de catalyser la réaction. Une fois la réaction terminée, ou lorsqu'il y a lieu de récupérer l'aldéhyde formé il est alors possible de mettre en contact la phase organique avec une phase aqueuse que l'on aura préalablement chargée en base forte, ce qui permettra de faire passer en phase aqueuse l'essentiel des phosphines ou équivalents ainsi que le métal de la colonne VIII. Il sera alors possible par acidification et mise en contact avec de l'amine grasse recyclée ou neuve, de refaire passer le complexe catalytique en phase organique et de réitérer la réaction de nombreuses fois.

Dans la suite de la description, on ne se référera plus qu'aux phosphines qui sont les plus utilisées les phosphines joueront le rôle de paradigme vis-à-vis des stibines et des arsines. Cet enseignement par l'exemple ne saurait être en aucun cas limitatif.

Les phosphines préférées sont les phosphines qui sont les meilleurs activateurs des métaux du groupe VIII, notamment les phosphines très basiques pKa > 7 telles que les phosphines de tricyclohexyle (ces pnictines basiques favorisent le complexe d'invention entre l'halogène et l'aryle). Il peut être toutefois plus intéressant notamment en raison de leur prix et en raison de leur stabilité, d'utiliser des phosphines de triaryle telles que les triphénylphosphines.

Les éléments ou métaux du groupe VIII préférés sont ceux de la colonne contenant le palladium (Ni, Pd, Pt), de préférence ceux qui font partie des métaux de la mine du platine. Ces métaux sont parfois désignés par l'expression "métal noble" dans la présente description. Le métal préféré est le palladium. Toutefois, les autres métaux, bien qu'étants moins actifs, peuvent présenter un intérêt significatif en raison du prix élevé du palladium.

Enfin, il convient de noter que l'on peut remplacer les phosphines par les phosphites.

Parmi les bases utilisables, il convient de citer les amines tertiaires (les autres amines quoique utilisables présentent l'inconvénient d'être susceptibles de donner des réactions parasites d'amidation).

Les amines tertiaires sont, en général des amines dont les 3 substituants sont des restes hydrocarbonés ayant de 1 à 20 atomes de carbone, tels que par exemple les radicaux alcoyle, cycloalcoyle ou aryle ou aralkyle.

De préférence les substituants ont de 1 à 10 atomes de carbone et avantageusement 1 à 4 atomes de carbone. Les radicaux cycliques tels que le radical cyclopentyle, cyclohexyle, cycloheptyle donnent des résultats particulièrement satisfaisants.

A titre d'exemple d'amine, on peut citer la triéthylamine, la tri-N-propylamine, la tri-N-butylamine, la diméthyléthylamine, la diméthylcyclohexylamine, l'éthyldiisopropylamine et la N-N-diéthylcyclohexylamine.

L'amine tertiaire peut également être une amine insaturée, de type aromatique comme par exemple la pyridine, les diverses picolines, la quinoléine. Toutefois, la base peut être également une base minérale forte ou un sel d'acide relativement faible ou un mélange des précédents. Bien entendu, les bases préférées sont celles qui répondent aux conditions de pKa ou de régulation de pH mentionnées précédemment.

C'est ainsi que les carbonates, les bicarbonates, les phosphates et les hydrogénophosphates, surtout ces derniers donnent de bons résultats. L'ion $HPO_4^-$- est particulièrement favorable. On peut également citer comme anions donnant de bons résultats les anions sulfates, les anions acétates, les anions trifluoroacétates.

Les pressions partielles en monoxyde de carbone et en hydrogène sont indépendamment de préférence supérieures à 1 bar ($10^5$ Pa) ou avantageusement encore supérieures à 10 bar ($10^6$ Pa). En général, on utilise des pressions partielles de 10 à 100 bar (dans la présente description, les zéros ne sont pas considérés comme des chiffres significatifs sauf si cela est spécifié autrement), de 1 à 10 MPa.

La pression que présente la phase vapeur peut varier très largement, en général elle va de 0,1 à 30 MPa (de 1 à 300 bar de préférence de 1 à 15 MPa, de 10 à 150 bar). La phase organique peut également contenir un solvant inerte dans les conditions de la réaction d'hydrocarbonylation.

Ainsi on peut utiliser des hydrocarbures aliphatiques ou cycloaliphatiques saturés tels que l'hexane, le cyclohexane, ou aromatiques tels que le benzène, le toluène, le xylène, des esters tels que le benzoate de méthyle, le téréphtalate de méthyle, l'adipate de diméthyle, le phtalate de dibutyle, le diacétate de tétraéthylèneglycol, des éthers, notamment de polyol ou cycliques tels que le tétrahydrofuranne ou le dioxane. Il convient également de noter que lorsque le substrat est liquide dans les conditions de la réaction, on peut utiliser un excès de substrat comme solvant.

En général, lorsqu'on utilise un solvant, la concentration en poids de substrat par volume de solvant constitutif de la phase organique est compris entre 5 et 50 %, de préférence entre 10 et 40 %.

Le rapport molaire dans la phase vapeur entre le monoxyde de carbone et l'hydrogène, varie généralement

entre 0,1 et 10, de préférence entre 0,5 et 5. Les zones optimales se trouvent au voisinage de l'équimolécularité. Toutefois il peut être intéressant d'augmenter la proportion d'hydrogène pour réduire la quantité d'acide formé et d'augmenter la quantité de monoxyde de carbone pour réduire la réaction de déshalogénation réductrice parasite.

Un autre but de la présente invention est de fournir un procédé d'hydrocarbonylation utilisant le réactif précédent. Ce but est atteint au moyen d'un procédé qui consiste à faire réagir un dérivé halogéné (ou pseudohalogéné) avec un réactif comportant :

a) une phase aqueuse contenant au moins une espèce chimique stable à caractère basique ;

b) une phase organique contenant un catalyseur comportant au moins un élément de la colonne VIII de la classification périodique des éléments, ledit élément de la colonne VIII de la classification périodique complexé avec au moins un agent de coordination (ou ligand).

c ) une phase vapeur contenant de l'hydrogène et du monoxyde de carbone.

Lesdits agents de coordination sont avantageusement des dérivés hydrocarbonés des éléments de la colonne V. Lesdits dérivés hydrocarbonés des éléments de la colonne V dérivent de l'azote telles les amines, du phosphore telles les phosphines, de l'arsenic telles les arsines et de l'antimoine telles les stibines ; ces composés seront, par analogie avec le terme de pnicture, désignés dans la présente description, sous le terme de pnictines. Ils sont avantageusement choisis parmi les dérivés hydrocarbonés du phosphore tels que les phosphines.

Avantageusement ledit catalyseur comporte comme pnictine, une trialkylphosphine, de préférence une triphénylphosphine. Ladite phosphine et ledit métal de la colonne VIII sont avantageusement sous forme de tétrakis phosphine métal.

La pression partielle de chacun de ces deux gaz étant au moins égale à $10^4$ Pa. La température de réaction préférée varie entre 100 et 200° C.

En pratique, on peut mettre en oeuvre le procédé selon l'invention en introduisant dans un autoclave inerte le composé halogéné la base, le catalyseur, la pnictine et le solvant, puis, après les purges habituelles, en alimentant l'autoclave avec une pression adéquate d'un mélange $CO/H_2$ ; le contenu de l'autoclave est ensuite porté sous agitation à la température convenable jusqu'à ce que l'absorption cesse. La pression dans l'autoclave peut être maintenue constante pendant la durée de la réaction grâce à une réserve de mélange gazeux, qui l'alimente à la pression choisie. En fin d'essai, l'autoclave est refroidi et dégazé. Le mélange réactionnel est récupéré.

Le procédé peut être mis en oeuvre en discontinu ou en continu comme indiqué précédemment en recyclant le catalyseur, la phosphine et la base.

Les substrats sont en général des composés voisins de ceux qui ont été décrits dans le Brevet US 3 960 932, mais il peut également s'agir de composés pseudo-halogénés c'est-à-dire de composés ou l'halogène est remplacé par un groupe qui peut partir sous la forme d'un anion du type tosylate ou triflate, voire trifluoroacétate.

Il convient de noter que parmi les halogénures, les fluorures donnent des résultats insignifiants.

Les substrats sont en général des halogènures ou pseudohalogénures d'aryle, le radical aryle pouvant être mono ou polycyclique, condensé ou non, hétéro ou homocyclique, ledit cycle ayant avantageusement 5 ou 6 chaînons, de préférence 6 chaînons.

La réaction présente le plus d'intérêt pour des composés de formule générale (I)

$$(Z)_n \text{ Ar-X}$$

dans laquelle X représente un atome de brome, un atome d'iode, un atome de chlore ou un pseudohalogène du type trifluoroacétoxyle ou tosyle ou triflate ;

où n est un entier de 0 à 3 y compris les bornes,

où Z représente indépendamment :

. un radical hydroxyle ; . un radical alkyle linéaire ou ramifié de 1 à 20 atomes de carbone ou un radical alkyle substitué par un ou plusieurs atomes de fluor et/ou de chlore, tels que par exemple les radical méthyle, éthyle, propyles, butyles, pentyles, hexyles, octyles, décyles, trifluorométhyle, difluorochlorométhyle, trichlorométhyle ; de préférence un radical alkyle inférieur substitué par 1 à 3 atomes de fluor et/ou de chlore ;

. un radical alcoxyle linéaire ou ramifié ayant 1 à 20 atomes de carbone ou un radical alcoxyle substitué par un ou plusieurs atomes de fluor et/ou de chlore ; de préférence un radical alcoxyle inférieur (ayant 1 à 4 atomes de carbone) ou un radical alcoxyle inférieur substitué par 1 ou 3 atomes de fluor et/ou de chlore, tel que par exemple les radicaux méthoxyle, éthoxyle, isopropoxyle, difluorochlorométhoxyle ou trichlorométhoxyle ;

. un radical cyclopentyle, cyclohexyle ou cyclooctyle ;

. un radical phényle ou un radical phényle substitué par 1 à 3 radicaux alkyles ou alcoxyle inférieurs, tel

que les radicaux xylyles, toluyles, méthoxyphényles, éthoxyphényles ;

.un radical alcoxycarbonyle ayant 2 à 11 atomes de carbone et de préférence 2 à 5 atomes de carbone tels que par exemple les radicaux méthoxycarbonyle, éthoxycarbonyle, propoxycarbonyle et butoxycarbonyle;

.un radical alcoxycarbonylalkyle dont la partie alcoxycarbonyle est telle que définie précédemment et la partie alkyle comporte 1 à 4 atomes de carbone ;

.un radical cyclopentyloxycarbonyle ou cyclohexyloxycarbonyle ;

.un radical phénoxycarbonyle ou méthylphénoxycarbonyle ;

.un radical alkylcarbonyloxyle ayant 2 à 11 atomes de carbone et de préférence 2 à 5 atomes de carbone, tel que par exemple les radicaux acétoxyle, propionyloxyle, butyryloxyle ;

.un radical cyclopentanoyloxyle ou cyclohexanoyloxyle ou diméthylbenzoyloxyle.

Les exemples qui suivent sont non limitatifs et illustrent l'invention.

## EXEMPLES

### Procédure générale

Dans un autoclave de 250 ml en acier inox, on charge :

$\phi Cl/Pd(OAc)_2/PCy_3/\phi CH_3/Base/H_2O$ 50 mmol 1matg, 5 mmol quantité suffisante pour 110 mmol 60 ml.

L'autoclave est purgé puis agité et porté à 180° C sous pression de CO (15 bar) et H2 (50 bar) maintenue constante.

Après 4 h de réaction, la masse réactionnelle est soutirée, décantée et analysée par CPG(chromatographie en phase gazeuse) et CLHP (chromatographie liquide haute performance).

On entend par :

RT = Le rendement sur le produit transformé

$$RT = \frac{\text{nombre de mole de produit final}}{\text{nombre de moles de produit transformé}}$$

RR = Le rendement sur le produit initial

$$RR = \frac{\text{nombre de moles de produit final}}{\text{nombre de moles de produit initial}}$$

TT = Le taux de transformation du produit de départ

$$TT = \frac{\text{nombre de mole de produit transformé}}{\text{nombre de moles de produit initial}}$$

### Exemple 1

| BASE | TT PhCl % | RR % PhCHO | RR % PhH | RR % $PhCO_2H$ |
|---|---|---|---|---|
| * $NEt_3$ | 7 | 3 | 1 | n.d. |
| $NEt_3$ | 71 | 31 | 21 | 8,5 |
| $Na_2HPO_4$ | 78 | 51 | 21 | 8 |
| NaOAC | 63 | 41 | 22 | 1 |

* essai mené en absence d'eau (milieu monophasique)

Charges d'un essai type

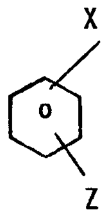

/Pd(OAc)$_2$ / $PR_3$ /$PhCH_3$ / Base / $H_2O$

50 mmol 1 matg 5 mmol qsp 25 ml 110 mmol 60 ml CO : 15 bar

$H_2$ : 50 bar ; t = 4 h

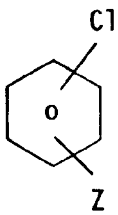

: $PR_3$ = $PCy_3$ ; t°C = 180° C

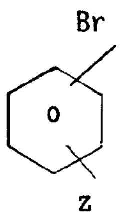

: $PR_3$ = $P\phi_3$ ; t°C = 150° C

Exemple 2

Rôle de la pression d'oxyde de carbone (pCO)
Substrat : Chlorobenzène (PhCl)
Base : Acétate de sodium
idem essai-type

| pCO bar | TT PhCl % | RT % PhCHO | PhH | $PhCO_2H$ |
|---|---|---|---|---|
| 0 | 100 | 0 | 100 | 0 |
| 15 | 69 | 57 | 31 | 2 |
| 50 | 26 | 76 | 15 | 5 |
| 100 | 12 | 85 | 10 | 8 |

Exemple 3

Influence des substituants

1°) Base : Acétate de sodium (NaOAc)
Substrat : p X - Ph - Cl
idem essai-type

| X | TT % | RT % PhCHO | PhH | $PhCO_2H$ |
|---|---|---|---|---|
| $CH_3O$ | 45 | 76 | 11 | 1 |
| H | 69 | 57 | 31 | 2 |
| $CF_3$ | 100 | 29 | 58 | n.d. |

2°) Substrat : pHO-PhBr
$PR_3$ : $PCy_3$
Base : $Na_2HPO_4$
t = 45 min
TT : 100 %
RT : parahydroxybenzaldéhyde : 5 %

Exemple 4

Influence de la pression partielle d'hydrogène

Substrat : PhCl
Base : NaOAc (acétate de sodium)
idem essai-type

**1°) Pression partielle de CO = 50 bar**

| p $H_2$ bar | TT PhCl % | RT % PhCHO | PhH | $PhCO_2H$ |
|---|---|---|---|---|
| 15 | 32 | 66 | 9 | 13 |
| 50 | 26 | 76 | 15 | 5 |
| 100 | 27 | 78 | 19 | 3 |

**2°) Pression partielle de CO = 15 bar**

| $pH_2$ bar | TT PhCl % | RT % PhCHO | PhH | $PhCO_2H$ |
|---|---|---|---|---|
| 0 | 90 | 2,5 | 1 | 93 |
| 15 | 70 | 71 | 15 | 12 |
| 50 | 69 | 57 | 31 | 2 |
| 100 | 64 | 55 | 47 | 1 |

Exemple 5

Influence de la température

* Substrat = ϕCl (ou PhCl) (idem essai-type ; base = acétate de sodium)

| T °C | TT PhCl % | RR % PhCHO | PhH | $PhCO_2H$ |
|---|---|---|---|---|
| 150 | 1,5 | 1 | 0,6 | 1 |
| 180 | 69 | 39 | 21 | 1 |
| 210 | 100 | 59 | 22 | 4 |

Exemple 6

Influence de la nature de la base

* Substrat : PhCl (charges et conditions opératoires : idem essai-type)

| BASE | TT PhCl % | PhCHO | RT % PhH | $PhCO_2H$ |
|---|---|---|---|---|
| NaOAc | 69 | 57 | 31 | 2 |
| NaOH | 100 | 20 | 65 | 0,5 |
| $Na_2CO_3$ | 100 | 36 | 50 | 0,5 |
| $Na_2HPO_4$ | 78 | 65 | 27 | 11 |
| $Et_3$ N | 71 | 44 | 30 | 12 |

* Substrat = PhBr (charges et conditions de l'essai-type)
Base : 55 mmol

| BASE | TT PhCl % | RT % PhCHO | PhH | $PhCO_2H$ |
|---|---|---|---|---|
| $Na_2HPO_4$ | 100 | 42 | 3 | 52 |
| $NEt_3$ | 100 | 38 | 7 | 43 |

Exemple 7

Influence de la nature du milieu

ϕ Cl / $Pd(OAc)_2$ / $PC_{y3}$ / E $t_3$ N / CO / $H_2$ / 180° C/4h 50 mmol 1 matg 5 mmol 110 mmol 15 bar 50 bar
Solvant : 30 ml

**1°) Milieu monophasique - sans eau**

| SOLVANT | TT PhCl % | PhCHO | RR % PhH | $PhCO_2H$ |
|---|---|---|---|---|
| $PhCH_3$ | 5 | 2,8 | 0,5 | 0,1 |
| o<br>o | 6 | 3,7 | 0,6 | 0,1 |

**2°) Milieu monophasique avec $H_2O$ (20 ml)**

| SOLVANT | TT PhCl % | RR % PhCHO | PhH | $PhCO_2H$ |
|---|---|---|---|---|
| 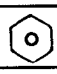 | 14 | 4 | 2,2 | 1,7 |

**3°) Milieu biphasique - $H_2O$ = 20 ml**

| SOLVANT | TT PhCl % | RR % PhCHO | PhH | $PhCO_2H$ |
|---|---|---|---|---|
| $PhCH_3$ | 48 | 32 | 11 | 1 |

Exemple 8

1°) Influence du pH initial de la phase aqueuse

| pH initial | pH final | TT % | Durée min | RT % PhCHO | RT % PhH | RT % $PhCO_2H$ |
|---|---|---|---|---|---|---|
| 4 | 1,5 | 70 | 100 | 63 | 3 | 18 |
| 5 | 2 | 100 | 60 | 70 | 4 | 21 |
| 6 | 2,3 | 100 | 60 | 74 | " | 21 |
| 7 | 4,2 | 100 | 20 | 77 | " | 15 |
| 8 | 4,4 | 100 | 20 | 72 | " | 21 |
| 9,1 | 4,2 | 100 | 20 | 74 | " | 17 |
| 12 | 6 | 100 | 10 | 62 | 11 | 3 |

$\phi Br$ / $Pd(OAc)_2$ / $PPh_3$ / $PhCH_3$ / $Na_2HPO_4$/ $H_2O$ / CO / $H_2$/ 150° C 50 mmol 1 matg 5 mmol 25 ml 55 mmol 60 ml 15 bar 15 bar

Remarques : pour les pH initiaux < 9,1 : ajustement par ajout de $H_3PO_4$

pHi = 9,1 : sans ajout

pHi = 12 : ajout de soude

Exemple 9

Essais avec trifluoroacétate de sodium $CF_3CO_2Na$

| Substrat | TT % | RT % ArCHO | RT % ArH | RT % $ArCO_2H$ |
|---|---|---|---|---|
| $\phi$ Cl | 68 | 54 | 38 | 4 |

ArCl / $Pd(OAc)_2$ / $PC_{y3}$ / $\phi CH_3$ / $CF_3$ $CO_2Na$/ $H_2O$ / CO / $H_2$ / 180°C/ 4h

50 mmol 1 matg 5 mmol 25 ml 110 mmol 60 ml 15 bar 50 bar

## Revendications

**1)** Réactif d'hydrocarbonylation de dérivés insaturés caractérisé par le fait qu'il comporte :

a) une phase aqueuse contenant au moins une espèce chimique stable à caractère basique,

b) une phase organique contenant un catalyseur comportant au moins un élément choisi parmi le nickel, le palladium et le platine de la colonne VIII de la classification périodique des éléments, ledit élément de la colonne VIII de la classification périodique complexé par au moins un agent de coordination (ou ligand) de préférence une pnictine,

c ) une phase vapeur contenant de l'hydrogène et du monoxyde de carbone ;

la pression partielle de chacun de ces deux gaz étant au moins égale à $10^4$ MPa.

**2)** Réactif selon la revendication 1 caractérisé par le fait que ladite espèce chimique a caractère basique est telle que le pKa de l'acide associé soit au plus égal à 10, de préférence au plus égal à 4.

**3)** Réactif selon l'une des revendications 1 et 2 caractérisé par le fait que ladite espèce chimique à caractère basique présente une basicité supérieure à celle des autres espèces présentes.

**4)** Réactif selon l'une des revendications 1 à 3 caractérisé par le fait que ladite espèce chimique présente une affinité telle que plus de la moitié de ladite espèce chimique se trouve en phase aqueuse.

**5)** Réactif selon l'une des revendications 1 à 4 caractérisé par le fait que ladite espèce chimique à caractère basique présente un pKa de l'acide associé au plus égal à 3, de préférence au plus égale à environ 2.

**6)** Réactif selon l'une des revendications 1 à 5 caractérisé par le fait que ledit élément du groupe VIII de la classification périodique des éléments est choisi parmi les métaux de la mine du platine et de leurs mélanges.

**7)** Réactif selon l'une des revendications 1 à 6 caractérisé par le fait que ledit pnictine est une phosphine, de préférence une trialkyl ou une triarylphosphine.

**8)** Réactif selon l'une des revendications 1 à 7 caractérisé par le fait que ladite phase organique comporte en outre un solvant choisi dans le groupe constitué par les solvants qui ne sont pas miscibles à l'eau en toute proportion, (inerte dans les conditions opératoires), dont le point de fusion est inférieur à 100° C environ.

**9)** Réactif selon l'une des revendications 1 à 7 caractérisé par le fait que la phase organique comporte comme solvant le substrat en excès.

**10)** Réactif selon l'une des revendications 1 à 9 caractérisé par le fait que le rapport molaire entre pnictine(s) et l'élément du groupe VIII de la classification périodiqe des éléments compris entre 2 et 1000 de préférence entre 4 et 1000.

**11)** Réactif selon l'une des revendications 1 à 10 caractérisé par le fait que la phase vapeur présente une pression totale comprise entre 0,1 et 30 MPa (1 à 300 bar) , de préférence entre 1 à 15 MPa (10 à 150 bar).

**12)** Réactif selon l'une des revendications 1 à 11, caractérisé par le fait que la pression partielle en hydrogène est au moins égale à 1 MPa (10 bar).

**13)** Réactif selon l'une des revendications 1 à 12 caractérisé par le fait qe la pression partielle en monoxyde de carbone est au moins égale à 1MPa (10 bar).

**14)** Réactif selon l'une des revendications 1 à 13 caractérisé par le fait que le rapport molaire hydrogène-/monoxyde de carbone dans la phase vapeur est compris entre 1/10 et 10.

**15)** Réactif selon l'une des revendications 1 à 14 caractérisé par le fait que le rapport des volumes de phase organique et de phase aqueuse est au plus égal à 10, à température ambiante.

**16)** Procédé de préparation d'aldéhydes insaturés par hydrocarbonylation caractérisé par le fait qu'il consiste à faire réagir un dérivé halogéné (ou pseudo-halogéné) avec un réactif comportant :

a) une phase aqueuse contenant au moins une espèce chimique stable à caractère basique ;

b) une phase organiqe contenant un pnictine organiqe et un élément du groupe VIII de la classification périodique des éléments ;

c) une phase vapeur contenant de l'hydrogène et du monoxyde de carbone.

La pression partielle de chacun de ces deux gaz étant au moins égale à $10^4$ Pa.

**17)** Procédé selon la revendication 16 caractérisé par le fait qe la réaction a lieu à une température comprise entre 100 et 200° C (1 seul chiffre significatif).

Office européen des brevets

# RAPPORT DE RECHERCHE EUROPEENNE

Numero de la demande

EP 91 40 3564

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 183 199 (MITSUI TOATSU)<br>* revendications 1-6 *<br>--- | 1 | C07C45/49<br>C07C47/52 |
| A | EP-A-0 352 166 (RHONE-POULENC CHIMIE)<br>* revendications 1,2 *<br>--- | 1 | |
| A | EP-A-0 163 234 (RUHRCHEMIE)<br>* revendication 1 *<br>--- | 1 | |
| A | FR-A-2 489 308 (JOHNSON, MATTHEY)<br>* revendication 1 *<br>----- | 1 | |

| DOMAINES TECHNIQUES RECHERCHES (Int. Cl.5 ) |
|---|
| C07C |

Le présent rapport a été établi pour toutes les revendications

| Lieu de la recherche | Date d'achèvement de la recherche | Examinateur |
|---|---|---|
| BERLIN | 01 AVRIL 1992 | PROBERT C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)